# EUROPÄISCHE PATENTSCHRIFT

(11) **EP 1 678 105 B1**
(45) Veröffentlichungstag und Bekanntmachung des Hinweises auf die Patenterteilung: **14.03.2007**
(21) Anmeldenummer: 04790914.8
(22) Anmeldetag: 27.10.2004
(51) Int. Cl.: C07C 11/08

(54) **VERFAHREN ZUR HERSTELLUNG VON 1-BUTEN**
METHOD FOR PRODUCING 1-BUTENE
PROCEDE DE FABRICATION DU 1-BUTENE

(30) Priorität: 27.10.2003 DE 10350045
(43) Veröffentlichungstag der Anmeldung: 12.07.2006
(73) Patentinhaber: BASF Aktiengesellschaft, 67056 Ludwigshafen (DE)
(72) Erfinder: SCHINDLER, Götz-Peter, 68219 Mannheim (DE); BRODHAGEN, Andreas, 67125 Dannstadt-Schauernheim (DE); JOHANN, Thorsten, 67117 Limburgerhof (DE); HILL, Thomas, 67071 Ludwigshafen (DE)
(74) Vertreter: Huhn, Michael
(86) Internationale Anmeldenummer: PCT/EP2004/012139
(87) Internationale Veröffentlichungsnummer: WO 2005/042450

(56) Entgegenhaltungen:
- US-A- 4 408 085
- US-A- 4 558 168

## Beschreibung

Die Erfindung betrifft ein Verfahren zur Herstellung von 1-Buten.

Butene können durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butenen, Butadien, Butinen, Methylallen, C₅- und höheren Kohlenwasserstoffen an.

Nachteilig an der Erzeugung von Buten im Crackprozess ist, dass zwangsläufig größere Mengen an Ethen oder Propen als Koppelprodukte anfallen.

Alternativ können Butene ausgehend von n-Butan durch katalytische Dehydrierung hergestellt werden (US 4 558 168). Nachteilig an diesen Verfahren ist jedoch, dass bei der katalytischen Dehydrierung von n-Butan neben 1-Buten auch 2-Buten und Butadien in größeren Mengen entsteht.

Aufgabe der Erfindung ist es, ein Verfahren zur Herstellung von 1-Buten aus n-Butan bereitzustellen, bei dem in möglichst geringem Umfang Koppelprodukte anfallen.

Gelöst wird die Aufgabe durch ein Verfahren zur Herstellung von 1-Buten aus n-Butan mit den Schritten
A) Bereitstellung eines n-Butan enthaltenden Einsatzgasstroms a;
B) Einspeisung des n-Butan enthaltenden Einsatzgasstroms a in mindestens eine Dehydrierzone und nicht-oxidative katalytische Dehydrierung von n-Butan, wobei ein Produktgasstrom b enthaltend n-Butan, 1-Buten, 2-Buten, Butadien, leichtsiedende Nebenbestandteile, Wasserstoff und gegebenenfalls Wasserdampf erhalten wird;
C) Abtrennung von Wasserstoff, der leichtsiedenden Nebenbestandteile und gegebenenfalls von Wasserdampf, wobei ein C₄-Produktgasstrom c im Wesentlichen bestehend aus n-Butan, 1-Buten, 2-Buten und Butadien erhalten wird;
D) Einspeisung des im Wesentlichen aus n-Butan, 1-Buten, 2-Buten und Butadien bestehenden C₄-Produktgasstroms c in eine Selektivhydrierungszone und Selektivhydrierung von Butadien zu 1- und/oder 2-Buten, wobei ein im Wesentlichen aus n-Butan, 1-Buten und 2-Buten bestehender Strom d erhalten wird;
E) Trennung des Stroms d in einen im wesentlichen aus 1-Buten bestehenden Strom e1 und einen im Wesentlichen aus 2-Buten und n-Butan bestehenden Rückführstrom e2 und Rückführung des im Wesentlichen aus 2-Buten und n-Butan bestehenden Rückführstroms e2 in die Dehydrierzone.

Das erfindungsgemäße Verfahren zeichnet sich durch eine besonders effektive Ausnutzung der Rohstoffe aus. So werden Verluste des Rohstoffs n-Butan durch Rückführung von nicht umgesetztem n-Butan in die Dehydrierung minimiert. n-Butan wird auf diese Weise praktisch vollständig umgesetzt. Bei der Dehydrierung gebildetes Butadien wird durch Selektivhydrierung in weiteres Wertprodukt umgewandelt, bei der Dehydrierung bzw. der Selektivhydrierung gebildetes 2-Buten wird in die Dehydrierung zurückgeführt. Es fallen somit weder Butadien noch 2-Buten als Koppelprodukte an. Ferner wird wegen der höheren Selektivität der nicht-oxidativen Butan-Dehydrierung gegenüber dem Steam-Cracken der Anfall von Ethen und Propen minimiert.

In einem ersten Verfahrensteil A wird ein n-Butan enthaltender Einsatzgasstrom a bereitgestellt. Üblicherweise wird dabei von an n-Butan reichen Gasgemischen wie liquefied petroleum gas (LPG) als Rohstoff ausgegangen. LPG enthält im Wesentlichen C₂-C₅-Kohlenwasserstoffe. Daneben enthält es auch Spuren von Methan und C₆⁺-Kohlenwasserstoffen. Die Zusammensetzung von LPG kann stark schwanken. Vorteilhafter Weise enthält das eingesetzte LPG mindestens 10 Gew.-% Butane.

Alternativ kann auch ein veredelter C₄-Kohlenwasserstoffstrom aus Crackern oder Raffinerien als Einsatzgasstrom a eingesetzt werden.

In einer Variante des erfindungsgemäßen Verfahrens umfasst die Bereitstellung des n-Butan enthaltenden Dehydrier-Einsatzgasstromes die Schritte
A1) Bereitstellung eines liquefied petroleum gas (LPG)-Stroms,
A2) Abtrennung von Propan und gegebenenfalls Methan, Ethan und C₅⁺-Kohlenwasserstoffen (hauptsächlich Pentane, daneben auch Hexane, Heptane, Benzol und Toluol) aus dem LPG-Strom, wobei ein Butane (n-Butan und Isobutan) enthaltender Strom erhalten wird,
A3) Abtrennung von Isobutan aus dem Butane enthaltenden Strom, wobei der n-Butan enthaltende Einsatzgasstrom erhalten wird, und gegebenenfalls Isomerisierung des abgetrennten lsobutans zu einem n-Butan/Isobutan-Gemisch und Rückführung des n-Butan/Isobutan-Gemischs in die Isobutan-Abtrennung.

Die Abtrennung von Propan und gegebenenfalls Methan, Ethan und C₅⁺-Kohlenwasserstoffen erfolgt beispielsweise in einer oder mehreren üblichen Rektifizierkolonnen. Beispielsweise können in einer ersten Kolonne Leichtsieder (Methan, Ethan, Propan) über Kopf und in einer zweiten Kolonne Schwersieder (C₅⁺-Kohlenwasserstoffe) am Kolonnensumpf abgetrennt werden. Es wird ein Butane (n-Butan und Isobutan) enthaltender Strom erhalten, aus dem Isobutan, beispielsweise in einer üblichen Rektifizierkolonne, abgetrennt wird. Der verbleibende, n-Butan enthaltende Strom wird als Einsatzgasstrom für die nachfolgende Butan-Dehydrierung eingesetzt.

Der abgetrennte Isobutan-Strom wird vorzugsweise einer Isomerisierung unterworfen. Dazu wird der Isobutan enthaltende Strom in einen Isomerisierungsreaktor eingespeist. Die Isomerisierung von Isobutan zu n-Butan kann wie in GB-A 2 018 815 beschrieben durchgeführt werden. Es wird ein n-Butan/Isobutan-Gemisch erhalten, das in die n-Butan/Isobutan-Trennkolonne eingespeist wird.

Der abgetrennte Isobutan-Strom kann auch einer weiteren Verwendung zugeführt werden, beispielsweise zur Herstellung von Methacrylsäure, Polyisobuten und Methyl-tert.-butylether eingesetzt werden.

In einem Verfahrensteil B wird der n-Butan enthaltende Einsatzgasstrom in eine Dehydrierzone eingespeist und einer nicht-oxidativen katalytischen Dehydrierung unterworfen. Dabei wird n-Butan in einem Dehydrierreaktor an einem dehydrieraktiven Katalysator teilweise zu 1-Buten und 2-Buten dehydriert, wobei auch Butadien gebildet wird. Daneben fallen Wasserstoff und in geringen Mengen Methan, Ethan, Ethen, Propan und Propen an. Je nach Fahrweise der Dehydrierung können außerdem Kohlenstoffoxide (CO, CO₂), Wasser und Stickstoff im Produktgasgemisch der nicht-oxidativen katalytischen n-Butan-Dehydrierung enthalten sein. Daneben liegt im Produktgasgemisch nicht umgesetztes n-Butan vor.

Die nicht-oxidative katalytische n-Butan-Dehydrierung kann mit oder ohne sauerstoffhaltigem Gas als Co-Feed durchgeführt werden.

Ein Merkmal der nicht-oxidativen Fahrweise gegenüber einer oxidativen Fahrweise ist das Vorhandensein von Wasserstoff im Austragsgas. Bei der oxidativen Dehydrierung wird kein freier Wasserstoff in wesentlichen Mengen gebildet.

Die nicht-oxidative katalytische n-Butan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung (analog dem Linde-Verfahren zur Propan-Dehydrierung), aber auch zwischen 3 und 8 bar bei Verwendung einer hohen Wasserdampfverdünnung (analog dem sogenannten "steam active reforming process" (STAR-Prozess) zur Dehydrierung von Propan oder Butan von Phillips Petroleum Co., siehe US 4,902,849, US 4,996,387 und US 5,389,342). Typische Katalysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein.

Die nicht-oxidative katalytische n-Butan-Dehydrierung kann auch, wie in Chem. Eng. Sci. 1992 b, 47 (9-11) 2313 beschrieben, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff und/oder Kohlenwasserstoffen in Gegenwart von Sauerstoff erzeugt werden. Gegebenenfalls kann zusätzlich ein Wasserstoff enthaltender Co-Feed zugemischt werden.

Die nicht-oxidative katalytische n-Butan-Dehydrierung kann mit oder ohne sauerstoffhaltigem Gas als Co-Feed in einem Hordenreaktor durchgeführt werden. Dieser enthält ein oder mehrere aufeinanderfolgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform, wobei mit sauerstoffhaltigem Co-Feed gearbeitet werden kann. In einer weiteren bevorzugten Ausführungsform wird die Dehydrierung in einem Hordenreaktor mit 3 Katalysatorbetten durchgeführt. Bei einer Fahrweise ohne sauerstoffhaltigem Gas als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherflächen oder durch Durchleiten durch mit heißen Brenngasen beheizte Rohre.

In einer bevorzugten Ausführungsform des erfindungsgemäßen Verfahrens wird die nicht-oxidative katalytische n-Butan-Dehydrierung autotherm durchgeführt. Dazu wird dem Reaktionsgasgemisch der n-Butan-Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Im allgemeinen wird die Menge des dem Reaktionsgasgemisch zugesetzten sauerstoffhaltigen Gases so gewählt, dass durch die Verbrennung von im Reaktionsgasgemisch vorhandenen Wasserstoff und gegebenenfalls von im Reaktionsgasgemisch vorliegenden Kohlenwasserstoffen und/oder von in Form von Koks vorliegendem Kohlenstoff die für die Dehydrierung des n-Butans benötigte Wärmemenge erzeugt wird. Im Allgemeinen beträgt die insgesamt zugeführte Sauerstoffmenge, bezogen auf die Gesamtmenge des Butans, 0,001 bis 0,5 mol/mol, bevorzugt 0,005 bis 0,2 mol/mol, besonders bevorzugt 0,05 bis 0,2 mol/mol. Sauerstoff kann entweder als reiner Sauerstoff oder als sauerstoffhaltiges Gas im Gemisch mit Inertgasen, beispielsweise in Form von Luft, eingesetzt werden. Die Inertgase und die resultierenden Verbrennungsgase wirken im Allgemeinen zusätzlich verdünnend und fördern damit die heterogen katalysierte Dehydrierung.

Der zur Wärmeerzeugung verbrannte Wasserstoff ist der bei der katalytischen n-Butan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff mit Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stelle des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemä-ßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100 °C, der Druck im letzten Katalysatorbett des Hordenreaktors im Allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen n-Butan-Dehydrierung sind die Katalysatoren gemäß den Beispiel 1, 2, 3 und 4 der DE-A 199 37 107.

Die n-Butan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben- Gegebenenfalls wird der Katalysator nach der Regenerierung mit einem wasserstoffhaltigen Gas reduziert.

Bei der n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der bevorzugten autothermen Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

Der Produktgasstrom der nicht-oxidativen autothermen n-Butan-Dehydrierung enthält typischerweise 0,1 bis 15 Vol.-% Butadien, 1 bis 15 Vol.-% 1-Buten, 1 bis 25 Vol.-% 2-Buten, 20 bis 70 Vol.-% n-Butan, 1 bis 70 Vol.-% Wasserdampf, 0 bis 10 Vol.-% leichtsiedende Kohlenwasserstoffe (Methan, Ethan, Ethen, Propan und Propen), 0,1 bis 40 Vol.-% Wasserstoff, 0 bis 70 Vol.-% Stickstoff und 0 bis 5 Vol.-% Kohlenstoffoxide.

In einem Verfahrensteil C werden die von den C₄-Kohlenwasserstoffen (n-Butan, isoButan, 1-Buten, cis-/trans-2-Buten, iso-Buten, Butadien) verschiedenen leicht siedenden Nebenbestandteile zumindest teilweise, vorzugsweise aber im wesentlichen vollständig aus dem Produktgasstrom der n-Butan-Dehydrierung abgetrennt, wobei ein C₄-Produktgasstrom c erhalten wird.

Der die Dehydrierzone verlassende Produktgasstrom b wird bevorzugt in zwei Teilströme aufgetrennt, wobei nur einer der beiden Teilströme den weiteren Verfahrensteilen C bis E unterworfen wird und der zweite Teilstrom in die Dehydrierzone zurückgeführt werden kann. Eine entsprechende Verfahrensweise ist in DE-A 102 11 275 beschrieben. Es kann jedoch auch der gesamte Produktgasstrom b der n-Butan-Dehydrierung den weiteren Verfahrensteilen C bis E unterworfen werden.

In einer Ausführungsform des erfindungsgemäßen Verfahrens wird im Verfahrensteil C zunächst Wasser aus dem Produktgasstrom b abgetrennt. Die Abtrennung von Wasser kann beispielsweise durch Auskondensieren durch Abkühlen und/oder Verdichten des Produktgasstroms b erfolgen und kann in einer oder mehreren Abkühlungs- und/oder Verdichtungsstufen durchgeführt werden. Die Wasserabtrennung wird üblicherweise durchgeführt, wenn die n-Butan-Dehydrierung autotherm durchgeführt oder unter Einspeisung von Wasserdampf isotherm (analog dem Linde- oder STAR-Prozess zur Dehydrierung von Propan) durchgeführt wird und folglich der Produktgasstrom b einen hohen Wasseranteil aufweist.

Die Abtrennung der leicht siedenden Nebenbestandteile aus dem Produktgasstrom kann durch übliche Trennverfahren wie Destillation, Rektifikation, Membranverfahren, Absorption oder Adsorption erfolgen.

Zur Abtrennung des im Produktgasstrom b der n-Butan-Dehydrierung enthaltenen Wasserstoffs kann das Produktgasgemisch, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist. Der so abgetrennte molekulare Wasserstoff kann bei Bedarf zumindest teilweise in der Dehydrierung eingesetzt oder aber einer sonstigen Verwertung zugeführt werden, beispielsweise zur Erzeugung elektrischer Energie in Brennstoffzellen eingesetzt werden.

Das in dem Produktgasstrom b der Dehydrierung enthaltene Kohlendioxid kann durch CO₂-Gaswäsche abgetrennt werden. Der Kohlendioxid-Gaswäsche kann eine gesonderte Verbrennungsstufe vorgeschaltet werden, in der Kohlenmonoxid selektiv zu Kohlendioxid oxidiert wird.

In einer bevorzugten Ausführungsform des erfindungsgemäßen Verfahrens werden die nicht kondensierbaren oder leicht siedenden Gasbestandteile wie Wasserstoff, Kohlenstoffoxide, die leicht siedenden Kohlenwasserstoffe (Methan, Ethan, Ethen, Propan, Propen) und gegebenenfalls Stickstoff in einem Absorptions-/Desorptions-Cyclus mittels eines hoch siedenden Absorptionsmittels abgetrennt, wobei ein C₄-Produktgasstrom c erhalten wird, der im wesentlichen aus den C₄-Kohlenwasserstoffen besteht. Im Allgemeinen besteht der C₄-Produktgasstrom c zu mindestens 80 Vol.-%, bevorzugt zu mindestens 90 Vol.-%, besonders bevorzugt zu mindestens 95 Vol.-% aus den C₄-Kohlenwasserstoffen.

Dazu wird in einer Absorptionsstufe der Produktgasstrom b - gegebenenfalls nach vorheriger Wasserabtrennung - mit einem inerten Absorptionsmittel in Kontakt gebracht und werden die C₄-Kohlenwasserstoffe in dem inerten Absorptionsmittel absorbiert, wobei mit C₄-Kohlenwasserstoffen beladenes Absorptionsmittel und ein die übrigen Gasbestandteile enthaltendes Abgas erhalten werden. In einer Desorptionsstufe werden die C₄-Kohlenwasserstoffe aus dem Absorptionsmittel wieder freigesetzt.

In der Absorptionsstufe eingesetzte inerte Absorptionsmittel sind im allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Die Absorption kann durch einfaches Durchleiten des Produktgasstroms b durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Geeignete Absorptionskolonnen sind z.B. Bodenkolonnen mit Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak^{®} 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

Geeignete Absorptionsmittel sind vergleichsweise unpolare organische Lösungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%. Geeignete Absorptionsmittel sind ferner Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption der C₄-Kohlenwasserstoffe wird das beladene Absorptionsmittel erhitzt und/oder auf einen niedrigeren Druck entspannt. Alternativ dazu kann die Desorption auch durch Strippung oder in einer Kombination von Entspannung, Erhitzen und Strippung in einem oder mehreren Verfahrensschritten erfolgen. Das in der Desorptionsstufe regenerierte Absorptionsmittel wird in die Absorptionsstufe zurückgeführt.

In einer Verfahrensvariante wird der Desorptionsschritt durch Entspannung und/oder Erhitzen des beladenen Absorptionsmittels durchgeführt.

Die Abtrennung C ist im Allgemeinen nicht ganz vollständig, so dass in dem C₄-Produktgasstrom - je nach Art der Abtrennung - noch geringe Mengen oder auch nur Spuren der weiteren Gasbestandteile, insbesondere der leicht siedenden Kohlenwasserstoffe, vorliegen können.

Der nach Abtrennung der Nebenbestandteile erhaltene C₄-Produktgasstrom c besteht im Wesentlichen aus n-Butan, 1-Buten, 2-Buten und Butadien. Im Allgemeinen enthält der Strom c 10 bis 80 Vol.-% n-Butan, 5 bis 40 Vol.-% 1-Buten, 10 bis 50 Vol.-% 2-Buten und 0 bis 40 Vol.-% Butadien. Bevorzugt enthält der Strom c 15 bis 65 Vol.% n-Butan, 10 bis 30 Vol.-% 1-Buten, 15 bis 45 Vol.-% 2-Buten und 5 bis 30 Vol.-% Butadien. Daneben kann der Strom c noch in geringen Mengen weitere Gasbestandteile enthalten wie Isobutan, Isobuten, C₅⁺-Kohlenwasserstoffe, Propan und Propen, im Allgemeinen in Mengen von 0 bis 10 Vol.%, bevorzugt von 0 bis 5 Vol.-%.

Die durch die Abtrennung der Nebenbestandteile bewirkte Volumenstromminimierung entlastet die nachfolgenden Verfahrensstufen.

In einem Verfahrensteil D wird der im Wesentlichen aus n-Butan, 1-Buten, 2-Buten und Butadien bestehende C₄-Produktgasstrom c in eine Selektivhydrierungszone eingespeist und wird eine Selektivhydrierung von Butadien zu 1-Buten und/oder 2-Buten durchgeführt.

Die Selektivhydrierung kann in an sich bekannter Weise in der Gasphase, Flüssigphase oder Rieselphase durchgeführt werden. Vorzugsweise wird die Selektivhydrierung in der Flüssig- oder Rieselphase mit fest angeordnetem Hydrierkatalysator durchgeführt. Als Hydrierkatalysatoren können Edelmetall-Träger-Katalysatoren eingesetzt werden, die Palladium, Platin, Silber, Gold, Rhodium, Ruthenium, Osmium oder Gemische dieser Metalle enthalten. Als Hydrierkatalysatoren sind Palladium enthaltende Trägerkatalysatoren besonders bevorzugt. Ein bevorzugt eingesetzter Hydrierkatalysator ist beispielsweise in EP-A 0 992 284 beschrieben. Dieser enthält Metalle der 8., 9. und 10. Gruppe des Periodensystems, insbesondere Ruthenium, Rhodium, Palladium und/oder Platin, auf einem Aluminiumoxid-Träger und daneben noch mindestens ein Metall der 11. Gruppe des Periodensystems, bevorzugt Kupfer und/oder Silber. Der Gehalt des Katalysators an Metall der 8., 9. oder 10. Gruppe des Periodensystems beträgt dabei im Allgemeinen 0,05 bis 2 Gew.-%, bevorzugt 0,1 bis 0,5 Gew.-%.

Die Selektivhydrierung kann in einem oder mehreren hintereinander geschalteten Reaktoren durchgeführt werden. Die Selektivhydrierung kann beispielsweise zweistufig ausgeführt werden. Die Temperatur liegt üblicher Weise im Bereich von 0°C bis 180°C und der Druck im Bereich von 2 bis 50 bar. In einer Ausführungsform wird die Selektivhydrierung bei einer Temperatur von 20 bis 90°C und einem Druck im Bereich von 5 bis 50 bar durchgeführt, wobei je Mol Butadien ein Mol Wasserstoff zugegeben wird.

Der die Selektivhydrierungszone verlassende Strom d besteht im Wesentlichen aus n-Butan, 1-Buten und 2-Buten. Im Allgemeinen enthält der Strom d 10 bis 80 Vol.-% n-Butan, 10 bis 60 Vol.-% 1-Buten, 15 bis 60 Vol.-% 2-Buten und 0 bis 5 Vol.-% Butadien. Bevorzugt enthält der Strom d 15 bis 65 Vol.-% n-Butan, 15 bis 45 Vol.-% 1-Buten, 20 bis 50 Vol.-% 2-Buten und 0 bis 1 Vol.-% Butadien. Daneben kann der Strom d noch in geringen Mengen weitere Gasbestandteile enthalten wie Isobutan, Isobuten, C₅⁺-Kohlenwasserstoffe, Propan und Propen, im Allgemeinen in Mengen von 0 bis 10 Vol.-%, bevorzugt von 0 bis 5 Vol.%.

In einem Verfahrensteil E wird der Strom d in einen im wesentlichen aus 1-Buten bestehenden Wertproduktstrom e1 und einen im Wesentlichen aus 2-Buten und n-Butan bestehenden Rückführstrom e2 aufgetrennt.

Die Auftrennung des im Wesentlichen aus 1-Buten, 2-Buten und n-Butan bestehenden Stroms d erfolgt im Allgemeinen in einer Destillationskolonne mit im Allgemeinen 30 bis 80, bevorzugt 40 bis 75 theoretischen Trennstufen. Geeignet sind beispielsweise Glockenbodenkolonnen, Füllkörperkolonnen, Packungskolonnen oder Trennwandkolonnen. Das Rücklaufverhältnis beträgt im Allgemeinen 10 bis 50. Die Destillation wird im Allgemeinen bei einem Druck von 5 bis 20 bar durchgeführt.

Im oberen Teil der Kolonne, vorzugsweise am Kolonnenkopf wird der im Wesentlichen aus 1-Buten bestehender Strom e1 abgezogen. Dieser enthält im Allgemeinen mindestens 90 Vol.%, vorzugsweise mindestens 95 Vol.-% 1-Buten und daneben bis zu 10 Vol.-%, vorzugsweise bis zu 5 Vol.-% weitere Bestandteile wie n-Butan und Isobuten.

Im unteren Teil der Kolonne, vorzugsweise im unteren Fünftel der Kolonne, besonders bevorzugt am Kolonnensumpf bis maximal 5 theoretische Böden oberhalb des Kolonnensumpfs, wird ein 2-Buten enthaltender Rückführstrom e2 abgezogen. Üblicherweise enthält der Strom e2 10 bis 80 Vol.-% 2-Buten und daneben 20 bis 90 Vol.-% n-Butan und 0 bis 5 Vol.-% 1-Buten. Der Strom e2 wird, gegebenenfalls nach Abtrennung eines Purgegasstroms, um die Aufpegelung von Hochsiedern zu vermeiden, in die n-Butan-Dehydrierung zurückgeführt.

### Beispiel

Ein Einsatzgasstrom a aus 140 946 t/a n-Butan wird zusammen mit einem im wesentlichen aus n-Butan und 2-Buten bestehenden Rückführstrom e2 (234 276 t/a; n-Butan 23,1%, cis-2-Buten 40,5%, trans-2-Buten 35,9%) in einen Dehydrierreaktor eingespeist und einer nicht-oxidativen katalytischen Dehydrierung unterzogen.

Es wird ein Produktgasstrom b der nachstehenden Zusammensetzung erhalten (alle nachstehenden %-Angaben sind Massen-%): n-Butan 15,9%, 1-Buten 22,7%, cis-2-Buten 18,9%, trans-2-Buten 23,0%, Butadien 17,6%.

Aus dem Produktgasstrom b wird Wasserstoff (8 639 t/a) abgetrennt, anschließend wird unter Zusatz von Wasserstoff (2 568 t/a) eine Selektivhydrierung durchgeführt.

Es wird ein im Wesentlichen aus n-Butan, 1-Buten und 2-Buten bestehender Strom d (386 429 t/a) der nachstehenden Zusammensetzung erhalten: n-Butan 16,1%, 1-Buten 34,4%, cis-2-Buten 26,2%, trans-2-Buten 23,3%.

In der nachfolgenden Destillation werden ein im Wesentlichen aus 1-Buten bestehender Wertproduktstrom e1 (136 496 t/a) und ein im Wesentlichen aus 2-Buten und n-Butan bestehenden Rückführstrom e2 (234 276 t/a) erhalten.

Der Wertproduktstrom e1 weist die folgende Zusammensetzung auf: n-Butan 3,3%, 1-Buten 96,6%, trans-2-Buten 0,1%.

Der Rückführstrom e2 weist die oben angegebene Zusammensetzung auf.

## Patentansprüche

1. Verfahren zur Herstellung von 1-Buten aus n-Butan mit den Schritten
A) Bereitstellung eines n-Butan enthaltenden Einsatzgasstroms a;
B) Einspeisung des n-Butan enthaltenden Einsatzgasstroms a in mindestens eine Dehydrierzone und nicht-oxidative, katalytische Dehydrierung von n-Butan, wobei ein Produktgasstrom b enthaltend n-Butan, 1-Buten, 2-Buten, Butadien, leichtsiedende Nebenbestandteile, Wasserstoff und gegebenenfalls Wasserdampf erhalten wird;
C) Abtrennung von Wasserstoff, der leichtsiedenden Nebenbestandteile und von Wasserdampf, wobei ein C₄-Produktgasstrom c enthaltend 10 bis 80 Vol.-% n-Butan, 5 bis 40 Vol.-% 1-Buten, 10 bis 50 Vol.-% 2-Buten, 0 bis 40 Vol.-% Butadien und 0 bis 10 Vol.-% weitere Gasbestandteile, erhalten wird;
D) Einspeisung des C₄-Produktgasstroms c in eine Selektivhydrierungszone und Selektivhydrierung von Butadien zu 1- und/oder 2-Buten, wobei ein Strom d enthaltend 10 bis 80 Vol.-% n-Butan, 10 bis 60 Vol.-% 1-Buten, 15 bis 60 Vol.-% 2-Buten, 0 bis 5 Vol.-% Butadien und 0 bis 10 Vol.-% weitere Gasbestandteile erhalten wird;
E) Trennung des Stroms d durch Destillation in einen im Wesentlichen aus 1-Buten bestehenden Wertproduktstrom e1 und einen im Wesentlichen aus 2-Buten und n-Butan bestehenden Rückführstrom e2 und Rückführung des im Wesentlichen aus 2-Buten und n-Butan bestehenden Rückführstroms e2 in die Dehydrierzone, wobei der Strom e1 mindestens 90 Vol.-% 1-Buten und der Strom e2 10 bis 80 Vol.-% 2-Buten, 20 bis 90 Vol.-% n-Butan und 0 bis 5 Vol.-% 1-Buten enthält.

2. Verfahren nach Anspruch 1, **dadurch gekennzeichnet, dass** die Dehydrierung von n-Butan als autotherme Dehydrierung durchgeführt wird.

3. Verfahren nach Anspruch 1 oder 2, **dadurch gekennzeichnet, dass** die Abtrennung der leichtsiedenden Nebenbestandteile in einem Absorptions-/Desorptions-Cyclus mittels eines hochsiedenden Lösungsmittels erfolgt.

## Claims

1. A process for preparing 1-butene from 1-butane, which comprises the steps
A) provision of an n-butane-comprising feed gas stream a;
B) introduction of the n-butane-comprising feed gas stream a into at least one dehydrogenation zone and nonoxidative catalytic dehydrogenation of n-butane to give a product gas stream b comprising n-butane, 1-butene, 2-butene, butadiene, low-boiling secondary constituents, hydrogen and possibly water vapor;
C) removal of hydrogen, the low-boiling secondary constituents and of water vapor to give a C₄ product gas stream c comprising from 10 to 80% by volume of n-butane, from 5 to 40% by volume of 1-butene, from 10 to 50% by volume of 2-butene, from 0 to 40% by volume of butadiene and from 0 to 10% by volume of further gas constituents;
D) introduction of the C₄ product gas stream c into a selective hydrogenation zone and selective hydrogenation of butadiene to 1- and/or 2-butene to give a stream d comprising from 10 to 80% by volume of n-butane, from 10 to 60% by volume of 1-butene, from 15 to 60% by volume of 2-butene, from 0 to 5% by volume of butadiene and from 0 to 10% by volume of further gas constituents;
E) separation of the stream d by distillation into a desired product stream e1 consisting essentially of 1-butene and a recycle stream e2 consisting essentially of 2-butene and n-butane and recirculation of the recycle stream e2 consisting essentially of 2-butene and n-butane to the dehydrogenation zone, the stream e1 comprising at least 90% by volume of 1-butene and the stream e2 comprising from 10 to 80% by volume of 2-butene, from 20 to 90% by volume of n-butane and from 0 to 5% by volume of 1-butene.

2. The process according to claim 1, wherein the dehydrogenation of n-butane is carried out as an autothermal dehydrogenation.

3. The process according to claim 1 or 2, wherein the removal of the low-boiling secondary constituents is carried out by means of a high-boiling solvent in an absorption/desorption cycle.

## Revendications

1. Procédé de préparation de 1-butène à partir de n-butane avec les étapes:
A) mise à disposition d'un flux a de gaz d'exploitation contenant du n-butane;
B) alimentation du flux a de gaz d'exploitation contenant du n-butane dans au moins une zone de déshydrogénation, et déshydrogénation catalytique non oxydative de n-butane, lors de laquelle un flux b de gaz de production contenant du n-butane, du 1-butène, du 2-butène, du butadiène, des éléments secondaires à bas point d'ébullition, de l'hydrogène et le cas échéant de la vapeur d'eau est obtenu;
C) séparation de l'hydrogène, des éléments secondaires à bas point d'ébullition et de la vapeur d'eau, dans laquelle un flux c de gaz de production de C₄ contenant 10 à 80 % en volume de n-butane, 5 à 40 % en volume de 1-butène, 10 à 50 % en volume de 2-butène, 0 à 40 % en volume de butadiène et 0 à 10 % en volume d'autres éléments gazeux est obtenu;
D) alimentation du flux c de gaz de production de C₄ dans une zone d'hydrogénation sélective et hydrogénation sélective de butadiène pour donner du 1-butène et/ou du 2-butène, dans laquelle un flux d contenant 10 à 80 % en volume de n-butane, 10 à 60 % en volume de 1-butène, 15 à 60 % en volume de 2-butène, 0 à 5 % en volume de butadiène et 0 à 10 % en volume d'autres éléments gazeux est obtenu;
E) séparation du flux d par distillation dans un flux e1 de produit de valeur constitué principalement de 1-butène et dans un flux e2 de recyclage constitué principalement de 2-butène et de n-butane et recyclage du flux e2 de recyclage constitué principalement de 2-butène et de n-butane dans la zone de déshydrogénation, le flux e1 contenant au moins 90 % en volume de 1-butène et le flux e2 contenant 10 à 80 % en volume de 2-butène, 20 à 90 % en volume de n-butane et 0 à 5 % en volume de 1-butène.

2. Procédé selon la revendication 1, **caractérisé en ce que** la déshydrogénation de n-butane est effectuée en tant que déshydrogénation autotherme.

3. Procédé selon la revendication 1 ou 2, **caractérisé en ce que** la séparation des éléments secondaires à bas point d'ébullition a lieu au moyen d'un solvant à haut point d'ébullition dans un cycle d'absorption/désorption.
